# EUROPEAN PATENT APPLICATION

(11) **EP 4 574 025 A1**
(43) Date of publication of application: **25.06.2025**
(21) Application number: 24222334.5
(22) Date of filing: 20.12.2024
(51) Int. Cl.: A61B 5/0205, A61B 5/145, A61B 5/024

(54) **APPARATUS FOR CONTROLLING BIOMETRIC DATA AND METHOD THEREOF**

(30) Priority: 22.12.2023 KR 20230190125; 18.11.2024 KR 20240164080
(71) Applicant: i-Sens, Inc., Seoul 06646 (KR)
(72) Inventor: LEE, Da Vid, 06646 Seoul (KR)
(74) Representative: Isarpatent

(57) **Abstract**

A method of controlling biometric data includes: obtaining first biometric data collected according to a first sampling rate; acquiring at least one first indicator value related to a first time interval of the first biometric data; acquiring at least one second indicator value related to a preset second time interval of the first biometric data; obtaining a first comparison result based on a difference value between the at least one first indicator value and the at least one second indicator value and threshold values set related to the first indicator value and the second indicator value; obtaining a first adjustment result for the first sampling rate based on the first comparison result; and determining a first variable sampling rate for the first biometric data based on the first adjustment result.

## Description

### BACKGROUND OF THE INVENTION

### Field of the Invention

Embodiments relate to an apparatus for controlling biometric data and a method thereof.

### Description of the Related Art

Recently, with the advancement of medical technology, various medical devices that are attached to a user's body have been developed and sold. The medical devices that are attached to the user's body may be usefully used to monitor biometric information or provide treatment by being attached to skins of patients with chronic diseases.

For example, chronic diseases such as diabetes need to be continuously managed. To monitor glucose of a diabetic patient, a medical device that is attached to the skin to measure the glucose may be used. Diabetes is characterized by almost no symptoms in the early stages. As the disease progresses, symptoms specific to diabetes such as overdrinking, polyphagia, polyuria, weight loss, general ennui, skin itching, and long-lasting wounds of hands and feet appear. As the diabetes progresses further, complications such as visual impairment, hypertension, kidney disease, stroke, periodontal disease, muscle cramps and neuralgia, and gangrene appear. In order to diagnose such diabetes and prevent the diabetes from progressing to complications, systematic glucose measurement and treatment needs to be performed in parallel.

For diabetics and people who have not progressed to diabetes but have more sugar detected in their blood than normal, many medical device manufacturers provide various types of glucose meters that may measure glucose.

For the glucose meter, a method in which a user measures glucose once by drawing blood from his/her fingertip and a method in which a user continuously measures glucose by attaching a glucose meter to his/her stomach, arm, etc., may be used.

Diabetics generally experience high and low glucose states, but emergencies occur when the diabetics are in a low glucose state, and diabetics may lose consciousness or even die when the diabetics remain in the low glucose state for a long time without sugar intake. Therefore, immediate detection of low glucose status is very important for diabetic patients. However, there is a limit to accurately detecting this with a blood glucose meter that measures glucose intermittently.

Recently, to overcome the limitation, a continuous glucose monitoring system (CGMS) that is inserted into a body to measures glucose levels at intervals of several minutes has been developed and used. The CGMS may continuously measure glucose by inserting a needle-shaped transdermal sensor into relatively less painful areas such as an abdomen and an arm to minimize users' pain and discomfort due to blood collection.

The CGMS is configured to include a sensor transmitter that is inserted into a user's skin to measure glucose in the body and transmit the measured glucose level, a terminal that outputs the obtained glucose level, etc.

Since the glucose is measured while the sensor transmitter is attached to the body and a part of the sensor is inserted into the body, the sensor inserted into the body may be recognized as foreign matters. That is, a biological reaction - for example, a hydration reaction - to a sensor may occur. This may cause a change in sensitivity (slope) at the initial stage of sensor insertion. This may decrease the accuracy of the sensor and make it difficult to maintain the service life. The changed slope may decrease the accuracy of the CGMS by obtaining an excessive or insufficient glucose level for the measured biosignal. In the past, in order to compensate for the change in sensitivity (slope), a method in which a simple sensitivity obtained by dividing a reference glucose level input by a user - a glucose level measured by a self-blood glucose monitoring system (BGMS) - by a current value of a sensor and a preset offset value are used or a method in which the sensitivity is determined through regression analysis on a reference glucose level and the related current value has been developed. However, there may be a problem in that the method does not respond sensitively to abnormal glucose levels or abnormal operation of the sensor, and thus provides inaccurate sensitivity and offset values.

### SUMMARY OF THE INVENTION

An embodiment may provide an apparatus for controlling biometric data for providing a sampling rate optimized for biometric data collection, and a method thereof.

The problem to be solved in the embodiment is not limited thereto, and the purpose or effect that may be grasped from the means or embodiment of the problem to be described below is included.

According to an embodiment of the present invention, a method of controlling biometric data may include: obtaining first biometric data collected according to a first sampling rate; acquiring at least one first indicator value related to a first time interval of the first biometric data; acquiring at least one second indicator value related to a second time interval of the first biometric data; obtaining a first comparison result based on (i) a difference value between the at least one first indicator value and the at least one second indicator value and (ii) threshold values set related to the first indicator value and the second indicator value; obtaining a first adjustment result for the first sampling rate based on the first comparison result; and determining a first variable sampling rate for the first biometric data based on the first adjustment result.

The first time interval may be arranged with priority over the second time interval.

The obtaining of the first comparison result based on the set threshold value may include: determining whether a size of the difference value is greater than the threshold value; and determining a sign of the difference value based on that the size of the difference value is greater than the threshold value.

The obtaining of the first adjustment result for the first sampling rate includes obtaining the first adjustment result as a adjustment result, which maintains the first sampling rate, based on that the size of the difference value is less than or equal to the threshold value.

The obtaining of the first adjustment result for the first sampling rate includes obtaining the first adjustment result as a adjustment result, which increases the first sampling rate, based on that the difference value is greater than the threshold value and the sign of the difference value is a first sign.

The obtaining of the first adjustment result for the first sampling rate includes obtaining the first adjustment result as a adjustment result, which decreases the first sampling rate, based on that the difference value is greater than the threshold value and the sign of the difference value is a second sign.

The obtaining the first adjustment result for the first sampling rate, at least one of an increment and a decrement of the first sampling rate is determined based on the size of the difference value.

The obtaining of the at least one second biometric data may include: acquiring at least one third indicator value related to the first time interval of each of the at least one second biometric data; acquiring at least one fourth indicator value related to the second time interval of each of the at least one second biometric data; obtaining a second comparison result based on (i) a difference value between the at least one third indicator value and the at least one fourth indicator value and (ii) threshold values set related to the third indicator value and the fourth indicator value; obtaining a second adjustment result for the first sampling rate based on the second comparison result; and determining a second variable sampling rate for the first biometric data based on the second adjustment result.

The method may include determining a third variable sampling rate for the first biometric data based on (i) the first variable sampling rate determined based on the first biometric data and (ii) at least one second variable sampling rate obtained based on the at least one second biometric data.

The determining of the third variable sampling rate includes determining the third variable sampling rate applied a weight related to each of the first variable sampling rate determined based on the first biometric data and the at least one second variable sampling rate determined based on the at least one second biometric data.

The determining of the third variable sampling rate, a set number of variable sampling rates among the first variable sampling rate determined based on the first biometric data and the at least one second variable sampling rate determined based on the at least one second biometric data may be selected according to a preset condition, and the third variable sampling rate may be determined based on the selected variable sampling rate.

The method may include controlling to collect the first biometric data according to the first variable sampling rate or the third variable sampling rate.

According to another embodiment of the present invention, an apparatus for controlling biometric data may include: a obtaining unit that obtains first biometric data collected according to a first sampling rate; an indicator acquisition unit that acquires at least one first indicator value related to a first time interval of the first biometric data and acquires at least one second indicator value related to a second time interval of the first biometric data; a comparison unit that obtains a first comparison result based on (i) a difference value between the at least one first indicator value and the at least one second indicator value and (ii) threshold values set related to the first indicator value and the second indicator value; a result obtaining unit that obtains a first adjustment result for the first sampling rate based on the first comparison result; and a sampling rate determination unit that determines a first variable sampling rate for the first biometric data based on the first adjustment result.

The obtaining unit may obtain at least one second biometric data, the indicator acquisition unit may acquire at least one third indicator value related to the first time interval of each of the at least one second biometric data and acquire at least one fourth indicator value related to the second time interval of each of the at least one second biometric data, the comparison unit may obtain a second comparison result based on (i) a difference value between the at least one third indicator value and the at least one fourth indicator value and (ii) threshold values set related to the third indicator value and the fourth indicator value, the result obtaining unit may obtain a second adjustment result for the first sampling rate based on the second comparison result, and the sampling rate determination unit may determine a second variable sampling rate for the first biometric data based on the second adjustment result.

The sampling rate determination unit may determine a third variable sampling rate for the first biometric data based on (i) the first variable sampling rate determined based on the first biometric data and (ii) at least one second variable sampling rate determined based on the at least one second biometric data, and the apparatus may further include a control unit that controls to collect the first biometric data according to the first variable sampling rate or the third variable sampling rate.

According to an embodiment, it is possible to provide the optimal sampling rate suitable for the user's usage status and environment.

In addition, it is possible to accurately identify the user's physical condition through the optimal sampling rate.

In addition, by providing the optimal sampling rate according to the user's activity status, it is possible for the apparatus for measuring biometric data to have the high power efficiency.

In addition, it is possible to provide the effect of improving the convenience and accuracy of patient monitoring.

Various and beneficial advantages and effects of the present inventive concept are not limited to the above description, and may be more easily understood in the course of describing the specific embodiments of the present inventive concept.

### BRIEF DESCRIPTION OF THE DRAWINGS

FIG. 1 is a diagram for describing a system for monitoring glucose according to an embodiment of the present invention.
FIG. 2 is a configuration diagram of an apparatus for controlling biometric data according to an embodiment of the present invention.
FIG. 3 is a flowchart of a method of controlling biometric data according to an embodiment of the present invention.
FIG. 4 is a flowchart illustrating in detail an embodiment of S340 and S350 of FIG. 3.
FIG. 5 is a first exemplary diagram for describing a process of obtaining a first variable sampling rate when there are multiple types of first and second indicator values for first biometric data.
FIG. 6 is a second exemplary diagram for describing a process of obtaining the first variable sampling rate when there are multiple types of the first and second indicator values.
FIG. 7 is a third exemplary diagram for describing a process of obtaining the first variable sampling rate when there are multiple types of the first and second indicator values.
FIG. 8 is a fourth exemplary diagram for describing a process of obtaining the first variable sampling rate when there are multiple types of the first and second indicator values.
FIG. 9 is a fifth exemplary diagram for describing a process of obtaining the first variable sampling rate when there are multiple types of the first and second indicator values.
FIG. 10 is a flowchart of a method of controlling biometric data according to an embodiment of the present invention.
FIG. 11 is a sixth exemplary diagram for describing a process of obtaining a third variable sampling rate using the first biometric data and the second biometric data.
FIG. 12 is a seventh exemplary diagram for describing a process of obtaining the third variable sampling rate using the first biometric data and the second biometric data.
FIG. 13 is an eighth exemplary diagram for describing a process of obtaining the third variable sampling rate using the first biometric data and the second biometric data.

### DETAILED DESCRIPTION OF THE INVENTION

The present invention may be variously modified and have several embodiments, and thus, specific embodiments will be illustrated in the drawings and be described. However, it is to be understood that the present invention is not limited to a specific embodiment, but includes all modifications, equivalents, and substitutions without departing from the scope and spirit of the present invention.

The terms including ordinal numbers such as 'second' and 'first' may be used to describe various components, but these components are not limited by these terms. The terms are used to distinguish one component from another component. For example, the second component may be named the first component and the first component may also be similarly named the second component, without departing from the scope of the present invention. A term 'and/or' includes a combination of multiple related described items or any one of the plurality of related described items.

It is to be understood that when one component is referred to as being "connected to" or "coupled to" another component, one component may be connected directly to or coupled directly to another component or be connected to or coupled to another component with the other component interposed therebetween. On the other hand, it is to be understood that when one component is referred to as being "connected directly to" or "coupled directly to" another component, it may be connected to or coupled to another component without the other component interposed therebetween.

The terms used herein are used only in order to describe specific embodiments rather than limiting the present invention. Singular forms include plural forms unless the context clearly indicates otherwise. It is to be understood that the term "include" or "have" used here specifies the presence of features, numbers, steps, operations, components, parts, or combinations thereof mentioned in the present specification, or combinations thereof, but does not preclude the presence or addition of one or more other features, numbers, steps, operations, components, parts, or combinations thereof.

Unless indicated otherwise, it is to be understood that all the terms used in the specification including technical and scientific terms have the same meaning as those that are generally understood by those who skilled in the art. Terms generally used and defined by a dictionary should be interpreted as having the same meanings as meanings within a context of the related art and should not be interpreted as having ideal or excessively formal meanings unless being clearly defined otherwise in the present specification.

Hereinafter, embodiments will be described in detail with reference to the accompanying drawings. The same reference numerals will be used to describe the same or similar components, independent of the reference numerals and an overlapped description of the same components will be omitted.

Hereinafter, a system for monitoring glucose will be described with reference to the drawings. The system for monitoring glucose described below may be a continuous glucose monitoring system that continuously or constantly measures and provides a concentration of glucose of a subject. In the present invention, a continuous glucose measurement system that continuously measures biometric information indicating a glucose level through a body attachment unit attached to a user's body for a certain period and transmits the measured biometric information to a communication terminal will be described.

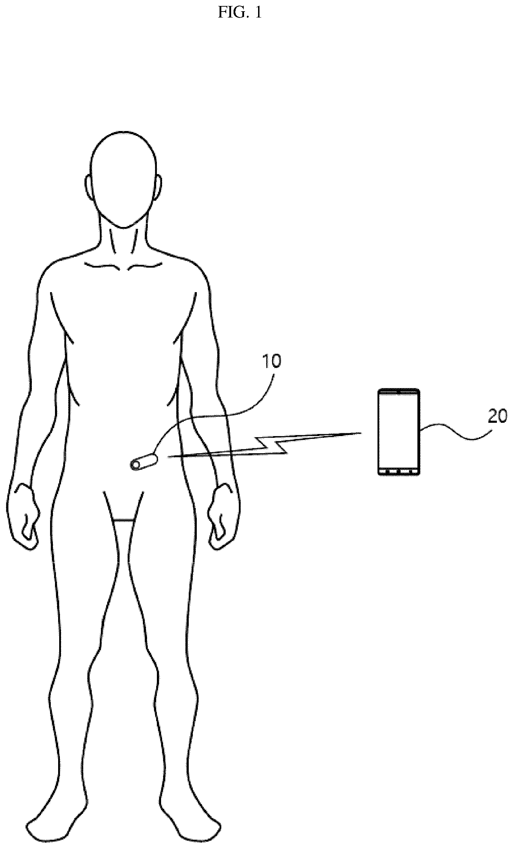

FIG. 1 is a diagram for describing a system for monitoring glucose according to an embodiment of the present invention.

Referring to FIG. 1, a system for monitoring glucose according to an embodiment of the present invention may include a sensor transmitter 10 and a receiver 20.

The sensor transmitter 10 may be attached to a human body B. When the sensor transmitter 10 is attached to the human body B, one end of the sensor transmitter 10 may be inserted into a skin to periodically measure a glucose concentration from a body fluid of the human body. In other words, the sensor may measure the glucose concentration in the body fluid through one end inserted into the skin. In this case, the body fluid of the human body may be interstitial fluid (ISF). The sensor transmitter 10 may convert the glucose concentration in the body fluid measured by the sensor into an electrical signal. The sensor transmitter 10 may convert the glucose concentration measured in the body fluid into the biometric information and output the biometric information.

To perform this function, the sensor transmitter 10 may include a sensor module and a transmitter. According to an embodiment, the sensor transmitter 10 may implement the sensor module and the transmitter as an integrated structure. In another embodiment, the sensor transmitter 10 may implement the sensor module and the transmitter as a separate structure. In this case, the sensor module may be attached to the human body and then coupled to the transmitter.

First, the sensor module may be a device that is inserted into the human body and measures the biometric information. The sensor module may be partially inserted into the skin and may measure the glucose concentration in the body fluid through the inserted portion.

Specifically, the sensor module may cause an electrochemical reaction with glucose contained in the interstitial fluid through a portion inserted into the skin. For example, the sensor module may include glucose oxidase, and contact the glucose contained in the interstitial fluid to allow the glucose oxidase to oxidize the glucose, thereby generating hydrogen peroxide (H2O2). The hydrogen peroxide may cause an oxidation-reduction reaction with an electrode included in the sensor module to generate current. The intensity of current generated in this process may correspond to the glucose concentration, so the sensor module may generate an electrical signal related to the glucose concentration in the interstitial fluid. The electrical signal may include a current value as an analog signal. In another embodiment, the electrical signal may include a voltage value as an analog signal.

Next, the transmitter may perform a function of processing the electrical signal generated by the sensor module. The transmitter may apply a set signal processing process to the electrical signal generated by the sensor module to generate the biometric information. The transmitter may transmit the generated biometric information to the receiver 20. To this end, the transmitter may include at least one of a control module, a communication module and a storage module.

The control module may control the overall configuration of the sensor transmitter 10 that includes the sensor module, the storage module, and the communication module.

According to an embodiment, the control module may process/manipulate an electrical signal. The control module may convert the electrical signal generated by the sensor module into the biometric information. The control module may convert the electrical signal, which is an analog signal, into the biometric information which is a digital signal. To this end, the control module may include an analog-to-digital converter (ADC). For example, the control module may amplify the electrical signal, and then convert the amplified electrical signal into the biometric information including a digital value through sampling, quantization, and coding steps. In addition, the control module may remove noise from the electrical signal before converting the electrical signal into the biometric information, if necessary. To this end, the control module may include a filter circuit, such as a low pass filter.

The communication module may transmit and receive various types of information to and from an external device.

According to an embodiment, the communication module may receive control information from the receiver 20. For example, the communication module may receive control information related to a transmission period of the biometric information from the receiver 20. The communication module may receive biometric information transmission request information from the receiver 20 or information on a transmission time (or time interval) of the biometric information.

According to an embodiment, the communication module may transmit the biometric information generated by the control module. The communication module may transmit the biometric information to the receiver 20. In addition, the communication module may transmit the biometric information to a server through a communication relay such as a router.

In addition to the embodiment, the communication module may transmit and receive various data related to the system for monitoring glucose to and from the external device.

According to an embodiment, the communication module may include a set communication interface to communicate with the external device. For example, the communication module may include a wired communication interface such as a USB communication module to perform wired communication with the receiver 20. Alternatively, the communication module may include a wireless communication interface, such as an infrared communication, NFC communication, Bluetooth, or WiFi communication module to perform wireless communication with the external device such as the receiver 20 or the server.

The storage module may store various types of information.

According to an embodiment, the storage module may store the biometric information generated by the control module. The storage module may store the biometric information or delete the stored biometric information under control of the control module.

According to an embodiment, the storage module may store control information. The storage module may store control information set at a factory. The storage module may store the control information received from the external device such as the receiver 20 or the server.

The receiver 20 may provide a user with various types of information based on the biometric information received from the sensor transmitter 10 and perform data processing accordingly.

According to an embodiment, the receiver 20 may obtain a glucose concentration contained in blood from the biometric information including the glucose information in the body fluid using a set algorithm. The set algorithm may be stored in the receiver 20. The receiver 20 may provide the obtained blood glucose concentration to the user through an output means such as a display.

According to an embodiment, the receiver 20 may generate various types of information based on the obtained glucose concentration in the blood. For example, the receiver 20 may generate trend information of the glucose concentration in the blood over a set time. Alternatively, the receiver 20 may generate warning information, such as low glucose warning or high glucose warning, based on the glucose concentration in the blood. The receiver 20 may provide the generated warning information to the user through the output means, such as the display or speaker.

In addition to the above embodiment, the receiver 20 may generate various types of information based on the biometric information and provide the generated information to a user.

In order to perform the above function, the receiver 20 may be implemented as various types of devices having a computational function, a storage function, and an output function. For example, the receiver 20 may include various devices, such as a smartphone, a mobile phone, a tablet PC, a desktop, and a laptop.

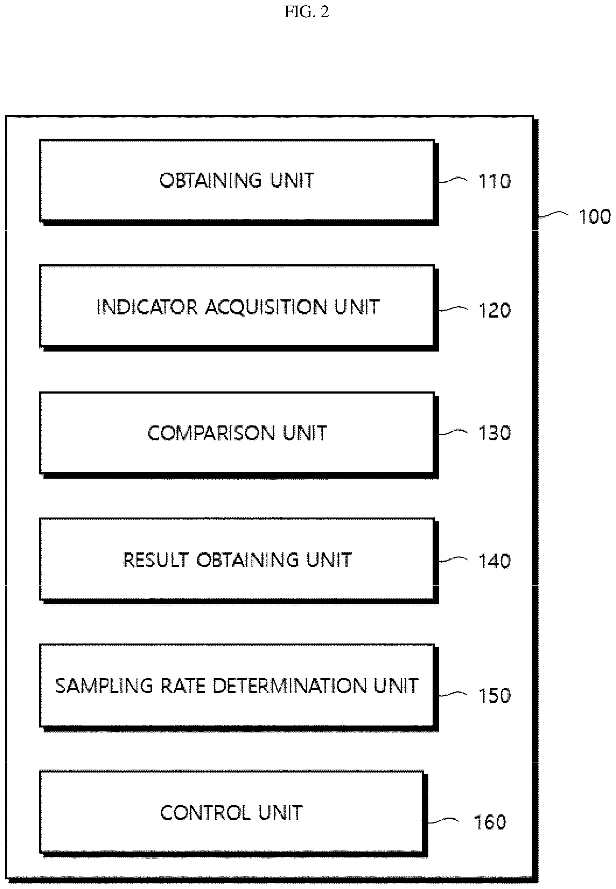

FIG. 2 is a configuration diagram of an apparatus for controlling biometric data according to an embodiment of the present invention.

The apparatus 100 for controlling biometric data described below refers to a device that may control a sampling rate for collecting biometric data. The apparatus 100 for controlling biometric data may control the sampling rate of the biometric data collected through the sensor. According to an embodiment, the apparatus 100 for monitoring biometric data may be implemented through the receiver 20. According to another embodiment, the apparatus 100 for controlling biometric data may be implemented through the sensor transmitter 10. According to another embodiment, the apparatus 100 for controlling biometric data may be implemented through the sensor transmitter 10 and the receiver 20. That is, some functions of the apparatus 100 for controlling biometric data may be implemented through the sensor transmitter 10, and some functions may be implemented through the receiver 20.

Referring to FIG. 2, the apparatus 100 for controlling biometric data according to an embodiment of the present invention may include a obtaining unit 110, an indicator acquisition unit 120, a comparison unit 130, a result acquisition unit 140, a sampling rate determination unit 150, and a control unit 160.

The obtaining unit 110 performs a function of obtaining biometric data. Here, the biometric data means biometric information of a subject such as a patient, and may include various types information such as a glucose level, a heart rate, and body temperature.

According to an embodiment, the obtaining unit 110 may obtain first biometric data collected according to a first sampling rate. Here, the first biometric data may be glucose data of a subject. Specifically, the first biometric data may be data indicating a measured glucose concentration in a body fluid of a subject.

According to an embodiment, the obtaining unit 110 may obtain at least one second biometric data. Here, the second biometric data may be different data from the first biometric data. The second biometric data may be different data from the glucose data of the subject. For example, the second biometric data may be heart rate data, exercise amount data, body temperature data, etc., of the subject. The second biometric data may be one type of biometric data. In addition, the second biometric data may be multiple biometric data for different types of information. For example, the obtaining unit 110 may obtain the heart rate data as the second biometric data, and as another example, the obtaining unit 110 may obtain the exercise amount data and the body temperature data as the second biometric data, respectively.

The indicator acquisition unit 120 performs a function of acquiring an indicator value for the biometric data. Here, the indicator value may mean a mathematical statistical value. For example, the indicator value may include a coefficient of variation, a standard deviation, a difference, dispersion, a relative standard deviation, an average, an arithmetic mean, a geometric mean, etc. In other words, the indicator acquisition unit 120 may acquire various mathematical statistical values related to the biometric data. Accordingly, the indicator value may include various mathematical statistical values in addition to the indicators described as examples above.

According to an embodiment, the indicator acquisition unit 120 may acquire at least one first indicator value related to a first time interval of the first biometric data. Here, the first time interval may be preset and adjusted. As an example, the first time interval may be set based on a current time point. As a specific example, the first time interval may be set as a 60-minute period starting from 2 hours before the current time point. When the current time point is 10:00 a.m., the indicator acquisition unit 120 may acquire a standard deviation of glucose data measured for 60 minutes from 8:00 a.m. to 9:00 a.m. as the first indicator value. When a plurality of first indicator values are acquired, the indicator acquisition unit 120 may acquire two or more statistical values, such as the standard deviation and the arithmetic mean of the glucose data measured for 60 minutes from 8:00 a.m. to 9:00 a.m. as the first indicator values, respectively. As another example, the first time interval may be set as a preset time interval within the time prior to the current time point.

According to an embodiment, the indicator acquisition unit 120 may acquire at least one second indicator value related to a preset second time interval of the first biometric data. Here, the second time interval may be preset and adjusted. As an example, the second time interval may be set based on the current time point. As a specific example, the second time interval may be an interval from the current time point to 30 minutes before, which is a preset time. When the current time point is 10:00 a.m., the second time interval may be set from 9:30 a.m. to 10:00 a.m., and the indicator acquisition unit 120 may acquire a second indicator value for glucose data measured for 30 minutes from 9:30 a.m. to 10:00 a.m. When the plurality of second indicator values are acquired, the indicator acquisition unit 120 may acquire two or more statistical values, such as the standard deviation and the arithmetic mean of the glucose data measured for 60 minutes from 8:00 a.m. to 9:00 a.m. as the second indicator values, respectively. As another example, the second time interval may be set as a preset time interval within the time prior to the current time point.

Meanwhile, the first indicator value and the second indicator value may be the same type of mathematical statistical values. For example, when the first indicator value is an arithmetic mean, the second indicator value may also be an arithmetic mean. As another example, when the first indicator value is an arithmetic mean and a geometric mean, the second indicator value may also be an arithmetic mean and a geometric mean.

Meanwhile, the first time interval of the first biometric data may be arranged with priority over the second time interval of the first biometric data. For example, when the first time interval is from 9:00 a.m. to 10:00 a.m., the second time interval may be arranged for a time after 10:00 a.m. When the first time interval is from 9:00 a.m. to 10:00 a.m., the second time interval is not arranged from 8:00 a.m. to 8:30 a.m.

According to an embodiment, the indicator acquisition unit 120 may acquire at least one third indicator value related to the first time interval of each of the at least one second biometric data. Here, the first time interval may be preset and adjusted. For example, the first time interval of the second biometric data may be substantially the same as the first time interval of the first biometric data. As another example, the first time interval of the second biometric data may be set according to set rules based on the first time interval of the first biometric data. For example, the first time interval of the second biometric data may be set to be earlier than the first time interval of the first biometric data by a set time. When the first time interval of the first biometric data is from 8:00 a.m. to 9:00 a.m., the first time interval of the second biometric data may be set from 7:50 a.m. to 8:50 a.m. These set rules may be set differently depending on the type of the second biometric data. As another example, the first time interval of the second biometric data may be set based on the current time point. As a specific example, the first time interval may be set as a 60-minute period starting from 2 hours before the current time point. In this case, the preset time of 60 minutes may be set differently depending on the type of biometric data.

According to an embodiment, the indicator acquisition unit 120 may acquire at least one fourth indicator value related to the preset second time interval of each of the at least one second biometric data. Here, the second time interval may be preset and adjusted. For example, the second time interval of the second biometric data may be substantially the same as the second time interval of the first biometric data. As another example, the second time interval of the second biometric data may be set according to the set rules based on the second time interval of the first biometric data. For example, the second time interval of the second biometric data may be set to be earlier than the second time interval of the first biometric data by a set time. When the second time interval of the first biometric data is from 09:30 a.m. to 10:00 a.m., the second time interval of the second biometric data may be set from 9:20 a.m. to 9:50 a.m. These set rules may be set differently depending on the type of the second biometric data. As another example, the second time interval of the second biometric data may be set based on the current time point. As a specific example, the first time interval may be an interval from the current time point to 30 minutes before, which is a preset time. In this case, the preset time of 30 minutes may be set differently depending on the type of biometric data.

Meanwhile, the third indicator value and the fourth indicator value may be the same type of mathematical statistical values. For example, when the third indicator value is an arithmetic mean, the fourth indicator value may also be an arithmetic mean. As another example, when the third indicator value is an arithmetic mean and a geometric mean, the fourth indicator value may also be an arithmetic mean and a geometric mean. Meanwhile, the third indicator value and the fourth indicator value may be mathematical statistical values of the same type as the first indicator value and the second indicator value, but are not limited thereto. The third indicator value and the fourth indicator value may be of different types from the first indicator value and the second indicator value.

Meanwhile, the first time interval of the second biometric data may be arranged with priority over the second time interval of the second biometric data. For example, when the first time interval is from 9:00 a.m. to 10:00 a.m., the second time interval may be arranged for a time after 10:00 a.m. When the first time interval is from 9:00 a.m. to 10:00 a.m., the second time interval is not arranged from 8:00 a.m. to 8:30 a.m.

The comparison unit 130 may perform a function of comparing the acquired indicator values according to the set rules to obtain the comparison result.

According to an embodiment, the comparison unit 130 may obtain a first comparison result based on a difference value between at least one first indicator value and at least one second indicator value and threshold values set related to the first indicator value and the second indicator value.

Specifically, the comparison unit 130 may determine whether a size of the difference value between the first indicator value and the second indicator value is greater than the threshold value. In addition, when the size of the difference value is greater than the threshold value, the comparison unit 130 may determine a sign of the difference value. According to the above determination process, the comparison unit 130 may output, as the first comparison result, any one of i) the size of the difference value is greater than the threshold value and the sign of the difference value is a first sign, ii) the size of the difference value is greater than the threshold value and the sign of the difference value is a second sign, and iii) the size of the difference value is less than or equal to the threshold value. Here, the first sign and the second sign are different signs, and when the first sign is (-), the second sign is (+), and when the first sign is (+), the second sign is (-). When there are multiple types of the first indicator values and the second indicator values, the first comparison result may be obtained according to the type of the indicator values, respectively. Meanwhile, when there are multiple types of the first indicator values and the second indicator values, it may be determined whether at least one difference value among the multiple types is greater than the threshold value. For example, the size of the difference value between the first indicator value and the second indicator value, which are the arithmetic mean, and the related threshold value may be compared, and the size of the difference value between the first indicator value and the second indicator value, which are the geometric mean, and the related threshold value may be compared.

According to an embodiment, the comparison unit 130 may obtain a second comparison result based on a difference value between at least one third indicator value and at least one fourth indicator value and threshold values set related to the third indicator value and the fourth indicator value.

Specifically, the comparison unit 130 may determine whether a size of the difference value between the third indicator value and the fourth indicator value is greater than the threshold value. In addition, when the size of the difference value is greater than the threshold value, the comparison unit 130 may determine a sign of the difference value. According to the above determination process, the comparison unit 130 may output, as the second comparison result, any one of i) the size of the difference value is greater than the threshold value and the sign of the difference value is a first sign, ii) the size of the difference value is greater than the threshold value and the sign of the difference value is a second sign, and iii) the size of the difference value is less than or equal to the threshold value. Here, the first sign and the second sign are different signs, and when the first sign is (-), the second sign is (+), and when the first sign is (+), the second sign is (-). When there are multiple types of the third indicator values and the fourth indicator values, the second comparison result may be obtained according to the type of the indicator values, respectively. In addition, when there are multiple types of the second biometric data, the second comparison result may be obtained depending on each type. Meanwhile, when there are multiple types of the third indicator values and the fourth indicator values, it may be determined whether at least one difference value among the multiple types is greater than the threshold value. For example, the size of the difference value between the third indicator value and the fourth indicator value, which are the arithmetic mean, and the related threshold value may be compared, and the size of the difference value between the third indicator value and the fourth indicator value, which are the geometric mean, and the related threshold value may be compared.

The result obtainng unit 140 may perform a function of obtaining an adjustment result for the first sampling rate according to the comparison result. Here, the first sampling rate may mean a sampling rate currently used to collect the first biometric data.

According to an embodiment, the result obtaining unit 140 may obtain the first adjustment result for the first sampling rate based on the first comparison result.

Specifically, the result obtaining unit 140 may obtain a first adjustment result that maintains the first sampling rate when the size of the difference value between the first indicator value and the second indicator value is less than or equal to the threshold value. The result obtaining unit 140 may obtain the first adjustment result that increases the first sampling rate when the difference value is greater than the threshold value and the sign of the difference value is the first sign. The result obtaining unit 140 may obtain the first adjustment result that decreases the first sampling rate when the difference value is greater than the threshold value and the sign of the difference value is the second sign.

Meanwhile, when there are multiple types of the first indicator values and the second indicator values, the first adjustment result may be obtained based on the first comparison result for each of the multiple types. For example, when both the first comparison result related to the arithmetic mean and the first comparison result related to the geometric mean have the first comparison result that the size of the difference value is less than or equal to the threshold value, the result obtaining unit 140 may obtain the first adjustment result that maintains the first sampling rate. On the other hand, when any of the first comparison results has a size of the difference value greater than the threshold value, the result obtaining unit 140 may obtain the first adjustment result that increases or decreases the first sampling rate. The result obtaining unit 140 may determine whether the difference value has the first sign or the second sign for a type of indicator values having the first comparison result that the difference value is greater than the threshold value to obtain the first adjustment result that increases or decreases the first sampling rate.

According to an embodiment, the result obtaining unit 140 may determine at least one of the increment and decrement of the first sampling rate based on the size of the difference value between the first indicator value and the second indicator value. The result obtaining unit 140 may determine at least one of the increment and decrement of the first sampling rate based on the set rules and the size of the difference value. For example, when at least one of the increment and decrement is set for each of a plurality of sections, the result obtaining unit 140 may determine at least one of the increment and decrement depending on which section the difference value is included in.

Meanwhile, when there are multiple types of indicator values having the first comparison result that the size of the difference value is greater than the threshold value, the increment or decrement may be determined using at least one of the increment or decrement obtained related to each of the multiple types of indicator values.

According to an embodiment, the result obtaining unit 140 may obtain a second adjustment result for the first sampling rate based on the second comparison result.

Specifically, the result obtaining unit 140 may obtain the second adjustment result that maintains the first sampling rate when the size of the difference value between the third indicator value and the fourth indicator value is less than or equal to the threshold value. The result obtaining unit 140 may obtain the second adjustment result that increases the first sampling rate when the difference value is greater than the threshold value and the sign of the difference value is the first sign. The result obtaining unit 140 may obtain the second adjustment result that decreases the first sampling rate when the difference value is greater than the threshold value and the sign of the difference value is the second sign.

Meanwhile, when there are multiple types of the third indicator values and the fourth indicator values, the first adjustment result may be obtained based on the second comparison result for each of the multiple types. For example, when both the second comparison result related to the arithmetic mean and the second comparison result related to the geometric mean have the second comparison result that the size of the difference value is less than or equal to the threshold value, the result obtaining unit 140 may obtain the first adjustment result that maintains the first sampling rate. On the other hand, when any of the second comparison results has the size of the difference value greater than the threshold value, the result obtaining unit 140 may obtain the first adjustment result that increases or decreases the first sampling rate. The result obtaining unit 140 may determine whether the difference value has the first sign or the second sign for a type of indicator values having the second comparison result that the size of the difference value is greater than the threshold value to obtain the first adjustment result that increases or decreases the first sampling rate.

According to an embodiment, the result obtaining unit 140 may determine at least one of the increment and decrement of the first sampling rate based on the size of the difference value between the third indicator value and the fourth indicator value. The result obtaining unit 140 may determine at least one of the increment and decrement of the first sampling rate based on the set rules and the size of the difference value. For example, when at least one of the increment and decrement is set for each of a plurality of sections, the result obtaining unit 140 may determine at least one of the increment and decrement depending on which section the difference value is included in.

Meanwhile, when there are multiple types of the indicator values having the second comparison result that the size of the difference value is greater than the threshold value, the increment or decrement of the first sampling rate may be determined using at least one of the increment or decrement obtained related to each of the multiple types of indicator values.

The sampling rate determination unit 150 performs a function of determining the variable sampling rate for the first biometric data based on the adjustment result.

According to an embodiment, the sampling rate determination unit 150 may determine a first variable sampling rate for the first biometric data based on the first adjustment result. The sampling rate determination unit 150 may determine the first variable sampling rate that maintains the first sampling rate according to the first adjustment result, increases the first sampling rate according to the increment, or decreases according to the decrement.

According to an embodiment, the sampling rate determination unit 150 may determine a second variable sampling rate for the first biometric data based on the second adjustment result.

According to an embodiment, the sampling rate determination unit 150 may determine a third variable sampling rate for the first biometric data based on the first variable sampling rate determined based on the first biometric data and at least one second variable sampling rate obtained based on at least one second biometric data.

According to an embodiment, the sampling rate determination unit 150 may apply related weights to the first variable sampling rate determined based on the first biometric data and at least one second variable sampling rate determined based on at least one second biometric data, respectively, to determine the third variable sampling rate. The weight may be set differently depending on the type of biometric data. For example, the sampling rate determination unit 150 may apply a high weight to the first variable sampling rate and a relatively low weight to the second variable sampling rate, and then acquire an average value of them, thereby determining the third variable sampling rate.

According to an embodiment, the sampling rate determination unit 150 may select a set number of variable sampling rates among the first variable sampling rate determined based on the first biometric data and at least one second variable sampling rate determined based on at least one second biometric data according to preset conditions, and determine the third variable sampling rate based on the selected variable sampling rate. For example, after all the first variable sampling rates and the second variable sampling rates are classified into one of categories of maintenance/increase/decrease, the average value of the variable sampling rates for the category with the largest number may be determined as the third variable sampling rate.

The control unit 160 performs a function of controlling to collect the first biometric data according to the determined variable sampling rate.

According to an embodiment, the control unit 160 may control to collect the first biometric data according to the first variable sampling rate or the third variable sampling rate. For example, when only the first variable sampling rate is determined by the sampling rate determination unit 150, the control unit 160 may control to collect the first biometric data according to the first variable sampling rate. On the other hand, when the third variable sampling rate is determined by the sampling rate determination unit 150, the control unit 160 may control to collect the first biometric data according to the third variable sampling rate.

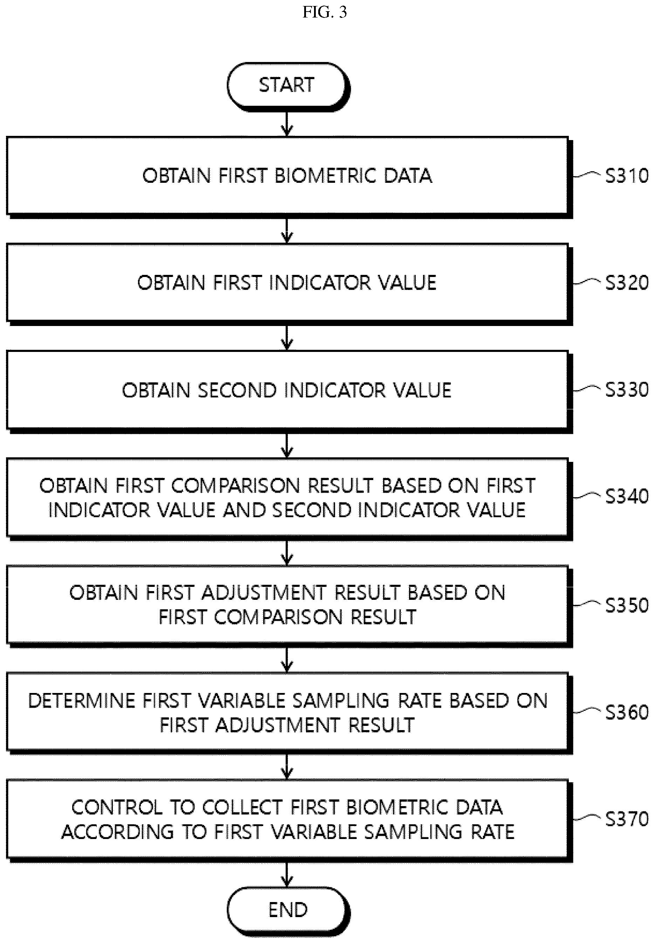

FIG. 3 is a flowchart of a method of controlling biometric data according to an embodiment of the present invention.

FIG. 3 is a flowchart of an embodiment of a method of controlling biometric data using only first biometric data.

First, the obtaining unit 110 may obtain the first biometric data collected according to the first sampling rate (S310).

Next, the indicator acquisition unit 120 may acquire at least one first indicator value related to the first time interval of the first biometric data (S320).

The indicator acquisition unit 120 may acquire at least one second indicator value related to the preset second time interval of the first biometric data (S330).

Next, the comparison unit 130 may obtain the first comparison result based on the difference value between at least one first indicator value and at least one second indicator value and the threshold values set related to the first indicator value and the second indicator value (S340). Specifically, the comparison unit 130 may determine whether the size of the difference value is greater than the threshold value. The comparison unit 130 may determine the sign of the difference value when the size of the difference value is greater than the threshold value.

Next, the result obtaining unit 140 may obtain the first adjustment result for the first sampling rate based on the first comparison result (S350). Specifically, the result obtaining unit 140 may obtain the first adjustment result that maintains the first sampling rate when the size of the difference value is less than or equal to the threshold value. The result obtaining unit 140 may obtain the first adjustment result that increases the first sampling rate when the difference value is greater than the threshold value and the sign of the difference value is the first sign. The result obtaining unit 140 may obtain the first adjustment result that decreases the first sampling rate when the difference value is greater than the threshold value and the sign of the difference value is the second sign. The result obtaining unit 140 may determine at least one of the increment and decrement of the first sampling rate based on the size of the difference value.

Next, the sampling rate determination unit 150 may determine the first variable sampling rate for the first biometric data based on the first adjustment result (S360).

The control unit 160 may control to collect the first biometric data according to the first variable sampling rate (S370).

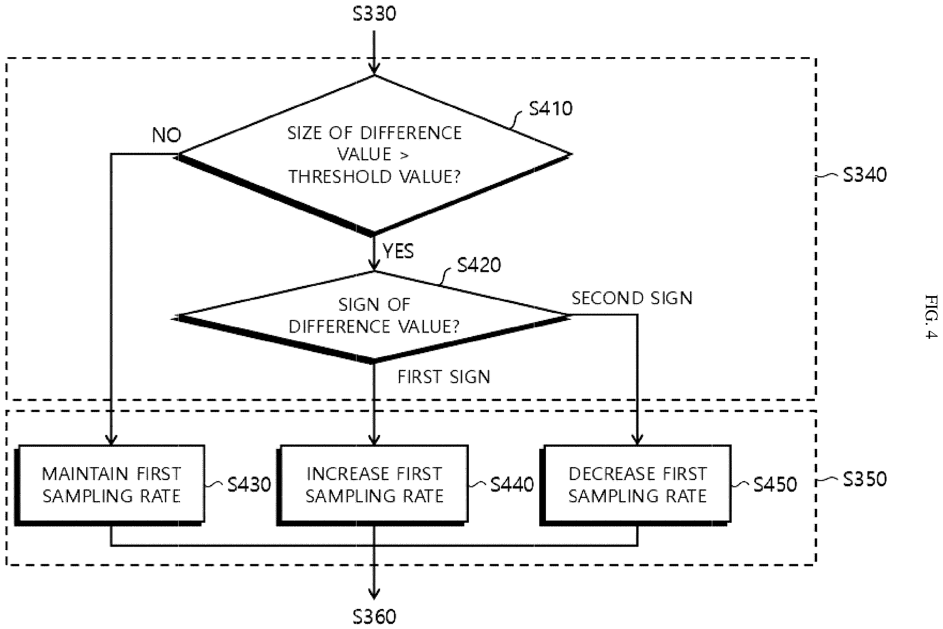

FIG. 4 is a flowchart illustrating in detail an embodiment of S340 and S350 of FIG. 3.

Referring to FIG. 4, the comparison unit 130 may determine whether the size of the difference value is greater than the threshold value (S410).

In addition, when the size of the difference value is greater than the threshold value, the comparison unit 130 may determine the sign of the difference value (S420).

Then, the result obtaining unit 140 may obtain the adjustment result based on the comparison result of the comparison unit 130.

First, the result obtaining unit 140 may obtain the first adjustment result that maintains the first sampling rate when the size of the difference value is less than or equal to the threshold value (S430).

Next, the result obtaining unit 140 may obtain the first adjustment result that increases the first sampling rate when the difference value is greater than the threshold value and the sign of the difference value is the first sign (S440).

Next, the result obtaining unit 140 may obtain the first adjustment result that decreases the first sampling rate when the difference value is greater than the threshold value and the sign of the difference value is the second sign (S450).

Meanwhile, the result obtaining unit 140 may determine at least one of the increment and decrement of the first sampling rate based on the size of the difference value.

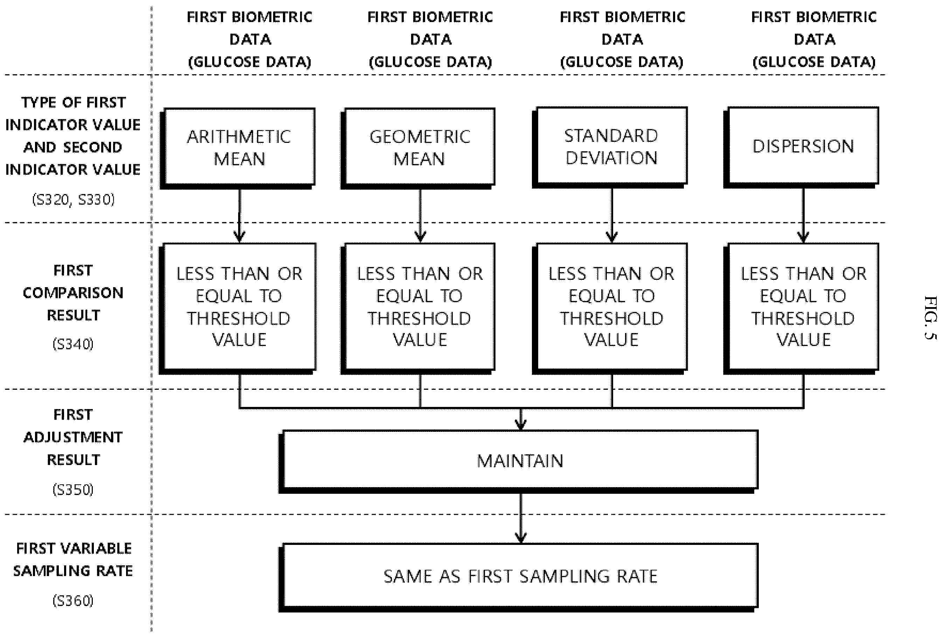

FIG. 5 is a first exemplary diagram for describing a process of obtaining the first variable sampling rate when there are multiple types of the first and second indicator values for the first biometric data.

FIG. 5 illustrates a case where the first comparison result related to each of the multiple types of indicator values is "less than or equal to the threshold value."

Referring to FIG. 5, the indicator acquisition unit 120 may obtain the first indicator value and the second indicator value related to the first biometric data for four types, respectively (S320 and S330). The indicator acquisition unit 120 may obtain the first indicator value and the second indicator value according to the arithmetic mean. The indicator acquisition unit 120 may obtain the first indicator value and the second indicator value according to the geometric mean. The indicator acquisition unit 120 may obtain the first indicator value and the second indicator value according to the standard deviation. The indicator acquisition unit 120 may obtain the first indicator value and the second indicator value according to the dispersion.

Then, the first comparison result may be obtained for each of the four types (S340). The comparison unit 130 may compare the first indicator value and the second indicator value according to the arithmetic mean with the threshold value set related to the arithmetic mean to obtain the first comparison result that the size of the difference value is less than or equal to the threshold value. The comparison unit 130 may compare the first indicator value and the second indicator value according to the geometric mean with the threshold value set related to the geometric mean to obtain the first comparison result that the size of the difference value is less than or equal to the threshold value. The comparison unit 130 may compare the first indicator value and the second indicator value according to the standard deviation with the threshold value set related to the standard deviation mean to obtain the first comparison result that the size of the difference value is less than or equal to the threshold value. The comparison unit 130 may compare the first indicator value and the second indicator value according to the dispersion with the threshold value set related to the dispersion to obtain the first comparison result that the size of the difference value is less than or equal to the threshold value. Therefore, four first comparison results may be obtained.

Since all the four first comparison results are "less than or equal to the threshold value," the result obtaining unit 140 may obtain the first adjustment result that maintains the existing first sampling rate (S350).

Then, the sampling rate determination unit 150 may determine the first variable sampling rate having the same value as the existing first sampling rate according to the first adjustment result (S360).

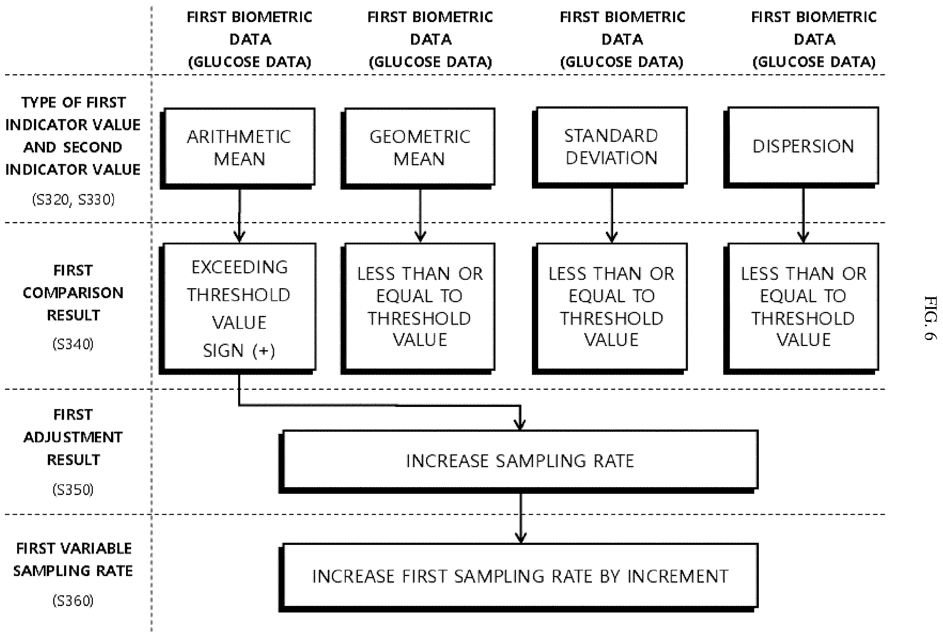

FIG. 6 is a second exemplary diagram for describing a process of obtaining the first variable sampling rate when there are multiple types of the first and second indicator values.

FIG. 6 illustrates a case where one of the first comparison results related to each of the multiple types of indicator values is "exceeding the threshold value."

Referring to FIG. 6, the indicator acquisition unit 120 may obtain the first indicator value and the second indicator value related to the first biometric data for four types, respectively (S320 and S330). The indicator acquisition unit 120 may obtain the first indicator value and the second indicator value according to the arithmetic mean. The indicator acquisition unit 120 may obtain the first indicator value and the second indicator value according to the geometric mean. The indicator acquisition unit 120 may obtain the first indicator value and the second indicator value according to the standard deviation. The indicator acquisition unit 120 may obtain the first indicator value and the second indicator value according to the dispersion.

Then, the first comparison result may be obtained for each of the four types (S340). The comparison unit 130 may compare the first indicator value and the second indicator value according to the arithmetic mean with the threshold value set related to the arithmetic mean to obtain the first comparison result that the size of the difference value exceeds the threshold value and the sign of the difference value is (+). The comparison unit 130 may compare the first indicator value and the second indicator value according to the geometric mean with the threshold value set related to the geometric mean to obtain the first comparison result that the size of the difference value is less than or equal to the threshold value. The comparison unit 130 may compare the first indicator value and the second indicator value according to the standard deviation with the threshold value set related to the standard deviation mean to obtain the first comparison result that the size of the difference value is less than or equal to the threshold value. The comparison unit 130 may compare the first indicator value and the second indicator value according to the dispersion with the threshold value set related to the dispersion to obtain the first comparison result that the size of the difference value is less than or equal to the threshold value. Therefore, four first comparison results may be obtained.

Since the first comparison result according to the arithmetic mean among the four comparison results is "exceeds the threshold value and the sign of the difference value is (+)", the result obtaining unit 140 may obtain the first adjustment result that increases the existing first sampling rate (S350). The result obtaining unit 140 may obtain the increment using the size of the difference value according to the arithmetic mean.

Then, the sampling rate determination unit 150 may determine the first variable sampling rate having a sampling rate related to the obtained increment compared to the existing first sampling rate according to the first adjustment result (S360).

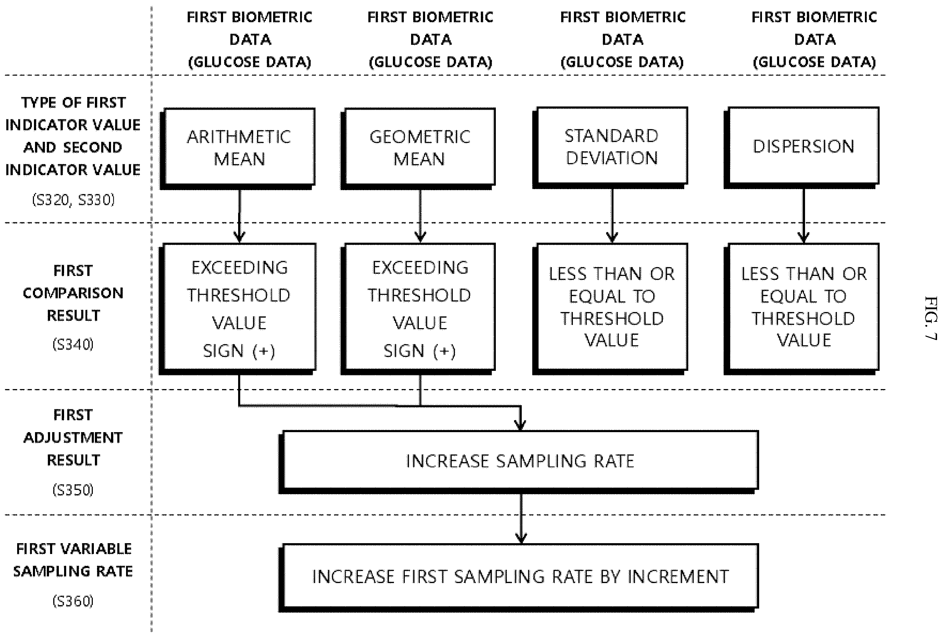

FIG. 7 is a third exemplary diagram for describing a process of obtaining the first variable sampling rate when there are multiple types of the first and second indicator values.

FIG. 7 illustrates a case where the plurality of first comparison results related to each of the multiple types of indicator values are "exceeding the threshold value."

Referring to FIG. 7, the indicator acquisition unit 120 may obtain the first indicator value and the second indicator value related to the first biometric data for four types, respectively (S320 and S330). The indicator acquisition unit 120 may obtain the first indicator value and the second indicator value according to the arithmetic mean. The indicator acquisition unit 120 may obtain the first indicator value and the second indicator value according to the geometric mean. The indicator acquisition unit 120 may obtain the first indicator value and the second indicator value according to the standard deviation. The indicator acquisition unit 120 may obtain the first indicator value and the second indicator value according to the dispersion.

Then, the first comparison result may be obtained for each of the four types (S340). The comparison unit 130 may compare the first indicator value and the second indicator value according to the arithmetic mean with the threshold value set related to the arithmetic mean to obtain the first comparison result that the size of the difference value exceeds the threshold value and the sign of the difference value is (+). The comparison unit 130 may compare the first indicator value and the second indicator value according to the geometric mean with the threshold value set related to the geometric mean to obtain the first comparison result that the size of the difference value exceeds the threshold value and the sign of the difference value is (+). The comparison unit 130 may compare the first indicator value and the second indicator value according to the standard deviation with the threshold value set related to the standard deviation mean to obtain the first comparison result that the size of the difference value is less than or equal to the threshold value. The comparison unit 130 may compare the first indicator value and the second indicator value according to the dispersion with the threshold value set related to the dispersion to obtain the first comparison result that the size of the difference value is less than or equal to the threshold value. Therefore, four first comparison results may be obtained.

Since the first comparison result according to the arithmetic mean and the first comparison result according to the geometric mean among the four first comparison results "exceed the threshold value and the sign of the difference value is (+)", the result obtaining unit 140 may obtain the first adjustment result using the first comparison result according to the arithmetic mean and the first comparison result according to the geometric mean. Then, the result obtaining unit 140 may obtain the first adjustment result that increases the existing first sampling rate according to the two first adjustment results (S350).

The increment may be obtained according to a set method. The result obtaining unit 140 may obtain the increment using the size of the difference value according to the arithmetic mean and the size of the difference value according to the geometric mean. Specifically, the result obtaining unit 140 may obtain the increment using the size of the difference value according to the arithmetic mean. For example, the result obtaining unit 140 may obtain the increment using an algorithm, a lookup table, etc., set related to the arithmetic mean of the first biometric data. The result obtaining unit 140 may obtain the increment using the size of the difference value according to the geometric mean. For example, the result obtaining unit 140 may obtain the increment using an algorithm, a lookup table, etc., set related to the geometric mean of the first biometric data. Then, the result obtaining unit 140 may obtain the final increment using the increment acquired based on the arithmetic mean and the increment acquired based on the geometric mean. For example, the result obtaining unit 140 may average the increment acquired based on the arithmetic mean and the geometric mean to obtain the final increment. As another example, the result obtaining unit 140 may apply a set weight to each of the increment acquired based on the arithmetic mean and the geometric mean and obtain the final increment through a process such as averaging or adding up them.

Then, the sampling rate determination unit 150 may determine the first variable sampling rate having a sampling rate related to the obtained increment compared to the existing first sampling rate according to the first adjustment result (S360). Specifically, the sampling rate determination unit 150 may determine the first variable sampling rate that increases the first sampling rate by the acquired final increment.

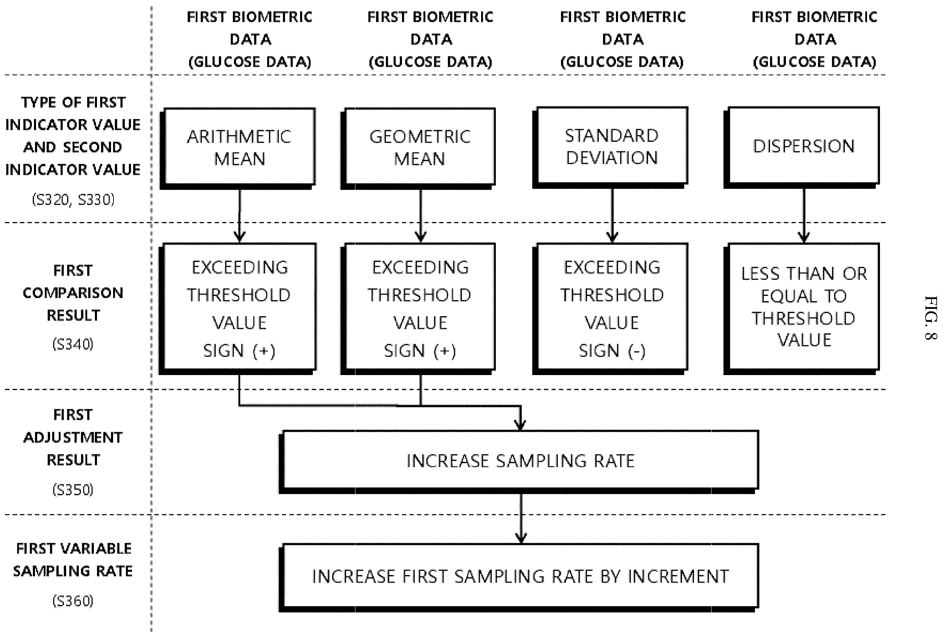

FIG. 8 is a fourth exemplary diagram for describing a process of obtaining the first variable sampling rate when there are multiple types of the first and second indicator values.

FIG. 8 illustrates a case where the types of the first comparison results related to each of the multiple types of indicator values are various.

Referring to FIG. 8, the indicator acquisition unit 120 may obtain the first indicator value and the second indicator value related to the first biometric data for four types, respectively (S320 and S330). The indicator acquisition unit 120 may obtain the first indicator value and the second indicator value according to the arithmetic mean. The indicator acquisition unit 120 may obtain the first indicator value and the second indicator value according to the geometric mean. The indicator acquisition unit 120 may obtain the first indicator value and the second indicator value according to the standard deviation. The indicator acquisition unit 120 may obtain the first indicator value and the second indicator value according to the dispersion.

Then, the first comparison result may be obtained for each of the four types (S340). The comparison unit 130 may compare the first indicator value and the second indicator value according to the arithmetic mean with the threshold value set related to the arithmetic mean to obtain the first comparison result that the size of the difference value exceeds the threshold value and the sign of the difference value is (+). The comparison unit 130 may compare the first indicator value and the second indicator value according to the geometric mean with the threshold value set related to the geometric mean to obtain the first comparison result that the size of the difference value exceeds the threshold value and the sign of the difference value is (+). The comparison unit 130 may compare the first indicator value and the second indicator value according to the standard deviation with the threshold value set related to the standard deviation to obtain the first comparison result that the size of the difference value exceeds the threshold value and the sign of the difference value is (-). The comparison unit 130 may compare the first indicator value and the second indicator value according to the dispersion with the threshold value set related to the dispersion to obtain the first comparison result that the size of the difference value is less than or equal to the threshold value. Therefore, four first comparison results may be obtained.

Then, the result obtaining unit 140 may selectively reflect some of the four first comparison results according to the set rules to obtain the first adjustment result (S350). For example, the result obtaining unit 140 may select a group having the same type of the first comparison result to obtain the first adjustment result. In FIG. 8, the first comparison result may be divided into three groups: `exceeding threshold value/sign (+)', `exceeding threshold/sign (-)', and `less than or equal to threshold value'. Among these, the group related to the `exceeding threshold value/sign (+)' includes the first comparison result according to the arithmetic mean and the first comparison result according to the geometric mean. Since the group related to the `exceeding threshold value/sign (+)' includes the largest number of first comparison results, the result obtaining unit 140 may obtain the first adjustment result that increases the existing first sampling rate based on the first comparison result according to the arithmetic mean and the first comparison result according to the geometric mean in the group related to the `exceeding threshold value/sign (+)'.

The increment may be obtained according to a set method. The result obtaining unit 140 may obtain the increment using the size of the difference value according to the arithmetic mean and the size of the difference value according to the geometric mean. Specifically, the result obtaining unit 140 may obtain the increment using the size of the difference value according to the arithmetic mean. For example, the result obtaining unit 140 may obtain the increment using an algorithm, a lookup table, etc., set related to the arithmetic mean of the first biometric data. The result obtaining unit 140 may obtain the increment using the size of the difference value according to the geometric mean. For example, the result obtaining unit 140 may obtain the increment using an algorithm, a lookup table, etc., set related to the geometric mean of the first biometric data. Then, the result obtaining unit 140 may obtain the final increment using the increment acquired based on the arithmetic mean and the increment acquired based on the geometric mean. For example, the result obtaining unit 140 may average the increment acquired based on the arithmetic mean and the geometric mean to obtain the final increment. As another example, the result obtaining unit 140 may apply a set weight to each of the increment acquired based on the arithmetic mean and the geometric mean and obtain the final increment through a process such as averaging or adding up the increment and the geometric mean.

Then, the sampling rate determination unit 150 may determine the first variable sampling rate having a sampling rate related to the obtained increment compared to the existing first sampling rate according to the first adjustment result (S360). Specifically, the sampling rate determination unit 150 may determine the first variable sampling rate that increases the first sampling rate by the acquired final increment.

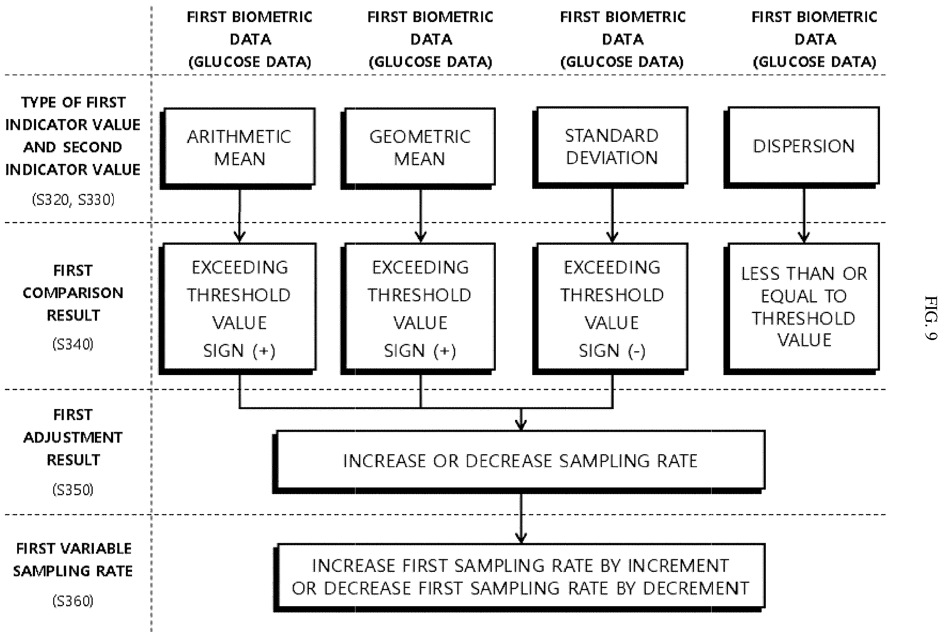

FIG. 9 is a fifth exemplary diagram for describing a process of obtaining the first variable sampling rate when there are multiple types of the first and second indicator values.

FIG. 9 illustrates a case where the types of the first comparison results related to each of the multiple types of indicator values are various.

Referring to FIG. 9, the indicator acquisition unit 120 may obtain the first indicator value and the second indicator value related to the first biometric data for four types, respectively (S320 and S330). The indicator acquisition unit 120 may obtain the first indicator value and the second indicator value according to the arithmetic mean. The indicator acquisition unit 120 may obtain the first indicator value and the second indicator value according to the geometric mean. The indicator acquisition unit 120 may obtain the first indicator value and the second indicator value according to the standard deviation. The indicator acquisition unit 120 may obtain the first indicator value and the second indicator value according to the dispersion.

Then, the first comparison result may be obtained for each of the four types (S340). The comparison unit 130 may compare the first indicator value and the second indicator value according to the arithmetic mean with the threshold value set related to the arithmetic mean to obtain the first comparison result that the size of the difference value exceeds the threshold value and the sign of the difference value is (+). The comparison unit 130 may compare the first indicator value and the second indicator value according to the geometric mean with the threshold value set related to the geometric mean to obtain the first comparison result that the size of the difference value exceeds the threshold value and the sign of the difference value is (+). The comparison unit 130 may compare the first indicator value and the second indicator value according to the standard deviation with the threshold value set related to the standard deviation to obtain the first comparison result that the size of the difference value exceeds the threshold value and the sign of the difference value is (-). The comparison unit 130 may compare the first indicator value and the second indicator value according to the dispersion with the threshold value set related to the dispersion to obtain the first comparison result that the size of the difference value is less than or equal to the threshold value. Therefore, four first comparison results may be obtained.

Then, the result obtaining unit 140 may selectively reflect some of the four first comparison results according to the set rules to obtain the first adjustment result (S350). For example, the result obtaining unit 140 may select all the first comparison results having the result of `exceeding the threshold value' to obtain the first adjustment result. In FIG. 9, the first comparison result having the result of `exceeding the threshold value' is the first comparison result according to the arithmetic mean, the first comparison result according to the geometric mean, and the first comparison result according to the standard deviation. Accordingly, the result obtaining unit 140 may obtain the first adjustment result that increases or decreases the existing first sampling rate based on the first comparison result according to the arithmetic mean, the first comparison result according to the geometric mean, and the first comparison result according to the standard deviation.

Determining the increase or decrease in the first sampling rate may be performed using the set rules. For example, the increase or decrease in the first sampling rate may be determined based on at least one of the increment and decrement acquired for each of the plurality of types. In FIG. 9, the result obtaining unit 140 may obtain the increment using the size of the difference value according to the arithmetic mean. For example, the result obtaining unit 140 may obtain the increment using an algorithm, a lookup table, etc., set related to the arithmetic mean of the first biometric data. The result obtaining unit 140 may obtain the increment using the size of the difference value according to the geometric mean. For example, the result obtaining unit 140 may obtain the increment using an algorithm, a lookup table, etc., set related to the geometric mean of the first biometric data. The result obtaining unit 140 may obtain the decrement using the size of the difference value according to the standard deviation. For example, the result obtaining unit 140 may obtain the increment using an algorithm, a lookup table, etc., set related to the standard deviation of the first biometric data. Then, the result obtaining unit 140 may subtract one decrement from two increments. In this case, the result obtaining unit 140 may obtain the first adjustment result that increases the first sampling rate when the subtraction result is positive, and obtain the first adjustment result that decreases the first sampling rate when the subtraction result is negative. The final decrement may be set based on the subtraction result, and may be similar to the method of acquiring the final increment or the final decrement described above.

Then, the sampling rate determination unit 150 may determine the first variable sampling rate having a sampling rate related to the obtained decrement compared to the existing first sampling rate according to the first adjustment result (S360). Specifically, the sampling rate determination unit 150 may determine the first variable sampling rate that decreases the first sampling rate by the acquired final decrement.

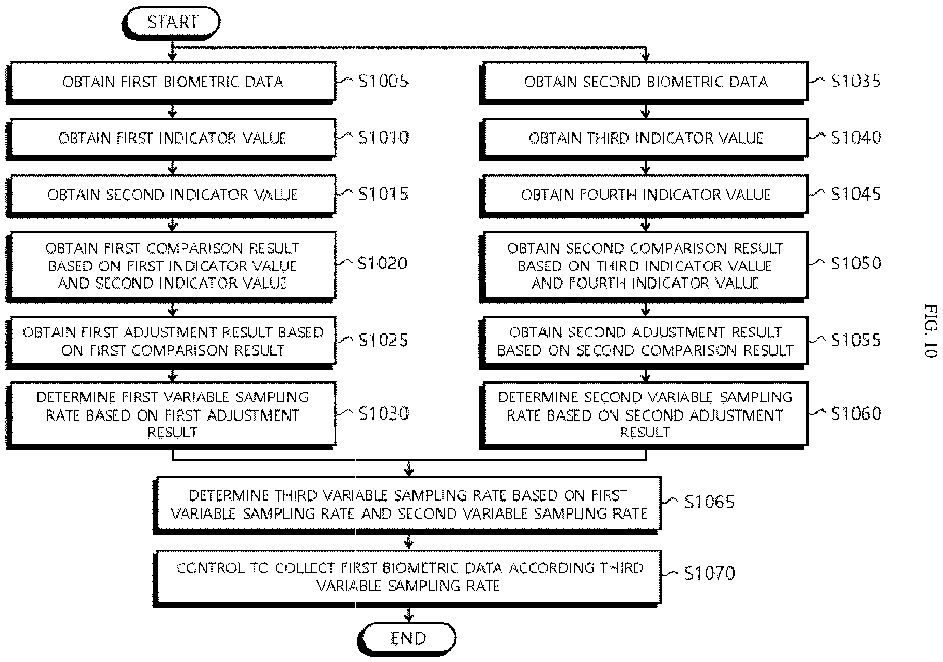

FIG. 10 is a flowchart of a method of controlling biometric data according to an embodiment of the present invention.

FIG. 10 is a flowchart of an embodiment describing a method of controlling biometric data using first biometric data and second biometric data.

First, the obtaining unit 110 may obtain the first biometric data collected according to the first sampling rate (S1005).

Next, the indicator acquisition unit 120 may acquire at least one first indicator value related to the first time interval of the first biometric data (S1010).

The indicator acquisition unit 120 may acquire at least one second indicator value related to the preset second time interval of the first biometric data (S 1015).

Next, the comparison unit 130 may obtain the first comparison result based on the difference value between at least one first indicator value and at least one second indicator value and the threshold values set related to the first indicator value and the second indicator value (S1020). Specifically, the comparison unit 130 may determine whether the size of the difference value is greater than the threshold value. The comparison unit 130 may determine the sign of the difference value when the size of the difference value is greater than the threshold value.

Next, the result obtaining unit 140 may obtain the first adjustment result for the first sampling rate based on the first comparison result (S1025). Specifically, the result obtaining unit 140 may obtain the first adjustment result that maintains the first sampling rate when the size of the difference value is less than or equal to the threshold value. The result obtaining unit 140 may obtain the first adjustment result that increases the first sampling rate when the difference value is greater than the threshold value and the sign of the difference value is the first sign. The result obtaining unit 140 may obtain the first adjustment result that decreases the first sampling rate when the difference value is greater than the threshold value and the sign of the difference value is the second sign. The result obtaining unit 140 may determine at least one of the increment and decrement of the first sampling rate based on the size of the difference value.

Next, the sampling rate determination unit 150 may determine the first variable sampling rate for the first biometric data based on the first adjustment result (S1030).

In addition, the obtaining unit 110 may obtain the second biometric data (S1035).

Next, the indicator acquisition unit 120 may acquire at least one third indicator value related to the first time interval of the second biometric data (S1040).

The indicator acquisition unit 120 may acquire at least one fourth indicator value related to the preset second time interval of the second biometric data (S1045).

Next, the comparison unit 130 may obtain the second comparison result based on the difference value between at least one third indicator value and at least one fourth indicator value and the threshold values set related to the third indicator value and the fourth indicator value (S1050). Specifically, the comparison unit 130 may determine whether the size of the difference value is greater than the threshold value. The comparison unit 130 may determine the sign of the difference value when the size of the difference value is greater than the threshold value.

Next, the result obtaining unit 140 may obtain the second adjustment result for the first sampling rate based on the second comparison result (S1055). Specifically, the result obtaining unit 140 may obtain the second adjustment result that maintains the first sampling rate when the size of the difference value is less than or equal to the threshold value. The result obtaining unit 140 may obtain the second adjustment result that increases the first sampling rate when the difference value is greater than the threshold value and the sign of the difference value is the first sign. The result obtaining unit 140 may obtain the second adjustment result that decreases the first sampling rate when the difference value is greater than the threshold value and the sign of the difference value is the second sign. The result obtaining unit 140 may determine at least one of the increment and decrement of the first sampling rate based on the size of the difference value.

Next, the sampling rate determination unit 150 may determine the first variable sampling rate for the second biometric data based on the second adjustment result (S1060).

Then, the sampling rate determination unit 150 may determine a third variable sampling rate for the first biometric data based on the first variable sampling rate determined based on the first biometric data and at least one second variable sampling rate obtained based on at least one second biometric data (S1065).

The control unit 160 may control to collect the first biometric data according to the third variable sampling rate (S1070).

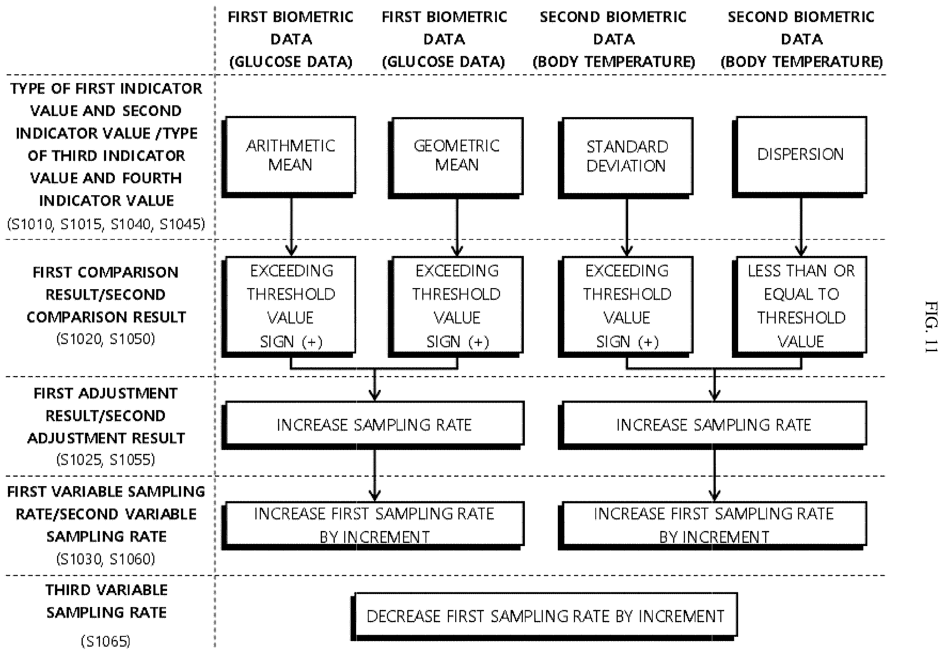

FIG. 11 is a sixth exemplary diagram for describing a process of obtaining a third variable sampling rate using the first biometric data and the second biometric data.

FIG. 11 illustrates a process of obtaining the third variable sampling rate using the first biometric data, which is glucose data, and the second biometric data, which is body temperature data.

Referring to FIG. 11, the indicator acquisition unit 120 may obtain the first indicator value and the second indicator value related to the glucose data, which is the first biometric data, for two types, respectively (S1010 and S1015). The indicator acquisition unit 120 may obtain the first indicator value and the second indicator value according to the arithmetic mean for the first biometric data. The indicator acquisition unit 120 may obtain the first indicator value and the second indicator value according to the geometric mean for the first biometric data. The indicator acquisition unit 120 may obtain the third indicator value and the fourth indicator value related to the body temperature data, which is the second biometric data, for two types, respectively (S1040 and S1045). The indicator acquisition unit 120 may obtain the third indicator value and the fourth indicator value according to the standard deviation for the second biometric data. The indicator acquisition unit 120 may obtain the third indicator value and the fourth indicator value according to the dispersion for the second biometric data.

Then, the comparison unit 130 may obtain the first comparison result for each of the two indicator types related to the first biometric data (S 1020). The comparison unit 130 may compare the first indicator value and the second indicator value according to the arithmetic mean with the threshold value set related to the arithmetic mean to obtain the first comparison result that the size of the difference value exceeds the threshold value and the sign of the difference value is (+). The comparison unit 130 may compare the first indicator value and the second indicator value according to the geometric mean with the threshold value set related to the geometric mean to obtain the first comparison result that the size of the difference value exceeds the threshold value and the sign of the difference value is (+).

The comparison unit 130 may obtain the second comparison result for each of the two indicator types related to the second biometric data (S1050). The comparison unit 130 may compare the third indicator value and the fourth indicator value according to the standard deviation with the threshold value set related to the standard deviation to obtain the second comparison result that the size of the difference value exceeds the threshold value and the sign of the difference value is (+). The comparison unit 130 may compare the third indicator value and the fourth indicator value according to the dispersion with the threshold value set related to the dispersion to obtain the second comparison result that the size of the difference value is less than or equal to the threshold value.

Then, the result obtaining unit 140 may obtain the first adjustment result according to the first biometric data, which is the glucose data, and the second adjustment result according to the second biometric data, which is the body temperature data (S1025 and S1055). First, since the first comparison result according to the arithmetic mean and the first comparison result according to the geometric mean, which are two first comparison results according to the first biometric data, are "exceeding the threshold value and the sign of the difference value is (+)," the result obtaining unit 140 may obtain the first adjustment result that increases the first sampling rate by using the first comparison result according to the arithmetic mean and the first comparison result according to the geometric mean. In addition, since the second comparison result according to the standard deviation among the two comparison results according to the second biometric data is "exceeding the threshold value and the sign of the difference value is (+)," the result obtaining unit 140 may obtain the second adjustment result that increases the existing first sampling rate.

The increment may be obtained according to a set method. Various embodiments for obtaining the increment have been described above, so a detailed description thereof will be omitted.

Then, the sampling rate determination unit 150 may determine the first variable sampling rate having a sampling rate related to the obtained increment compared to the existing first sampling rate according to the first adjustment result (S1030). Then, the sampling rate determination unit 150 may determine the second variable sampling rate having the sampling rate related to the obtained increment compared to the existing first sampling rate according to the second adjustment result (S1060).

The sampling rate determination unit 150 may determine the third variable sampling rate having a higher sampling rate than the first sampling rate based on the first variable sampling rate and the second variable sampling rate (S1065). For example, the sampling rate determination unit 150 may obtain the third variable sampling rate using the average value of the first variable sampling rate and the second variable sampling rate or by using the weights applied to the first variable sampling rate and the second variable sampling rate.

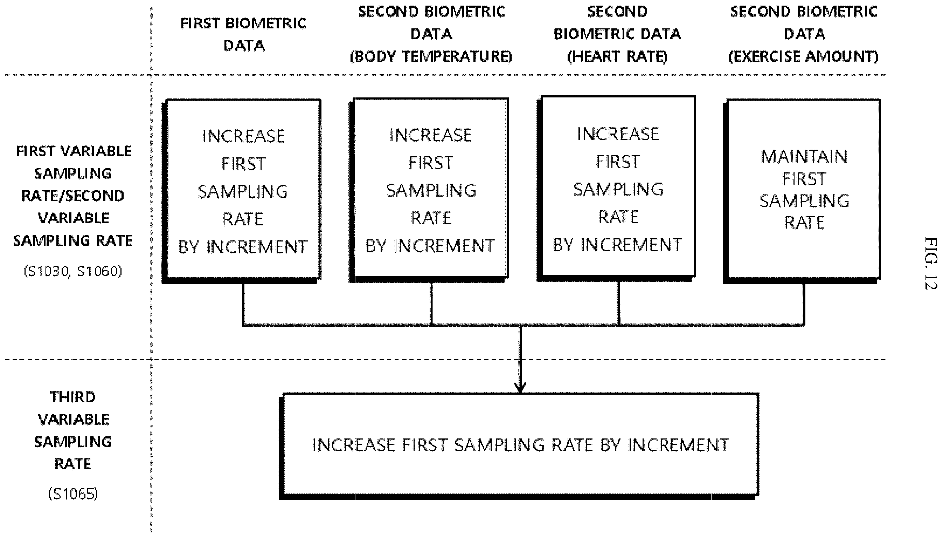

FIG. 12 is a seventh exemplary diagram for describing a process of obtaining the third variable sampling rate using the first biometric data and the second biometric data.

FIG. 12 illustrates a case in which one first biometric data and three second biometric data are used. Referring to FIG. 12, in order to obtain the third variable sampling rate, the first biometric data, which is the glucose data, the second biometric data, which is the body temperature data, the third biometric data, which is the heart rate data, and the fourth biometric data, which is the exercise amount data, may be used.

The process of obtaining the first variable sampling rate and the second variable sampling rate may be obtained using various embodiments described above, and a detailed description thereof will be omitted (S1030 and S 1060).

The first variable sampling rate related to the glucose data has a value that increases the first sampling rate by the obtained increment. The second variable sampling rate related to the body temperature data has a value that increases the first sampling rate by the obtained increment. The second variable sampling rate related to the heart rate data has a value that increases the first sampling rate by the obtained increment. The second variable sampling rate related to the exercise amount data has the same value as the first sampling rate.

In this case, the sampling rate may select the variable sampling rate according to the set rules, and determine the third variable sampling rate based on the selected variable sampling rate. For example, a type that accounts for more than half of the first variable sampling rate and the second variable sampling rate may be selected. In FIG. 12, there are the types that increase the first sampling rate, such as the first variable sampling rate according to the glucose data, the second variable sampling rate according to the body temperature data, and the second variable sampling rate according to the heart rate data. Therefore, the sampling rate determination unit 150 may determine the third variable sampling rate using the related three variable sampling rates (S1065).

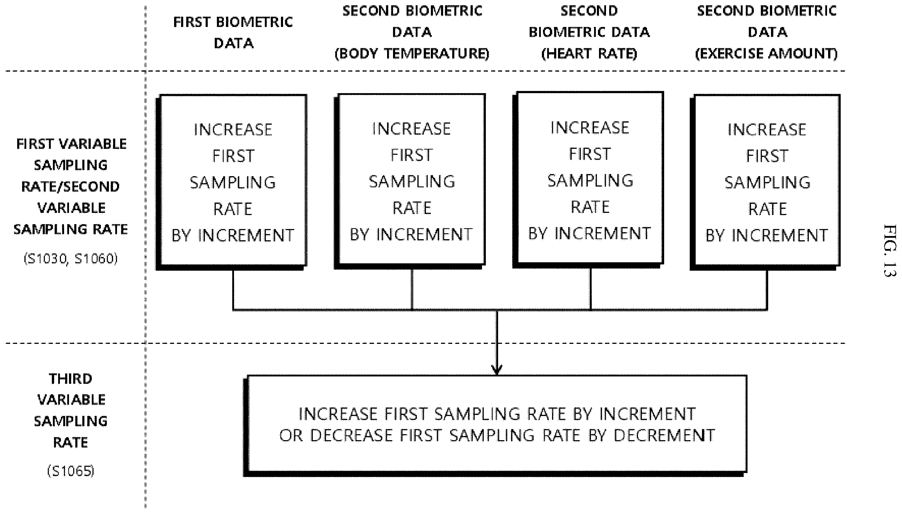

FIG. 13 is an eighth exemplary diagram for describing a process of obtaining the third variable sampling rate using the first biometric data and the second biometric data.

FIG. 13 illustrates a case in which one first biometric data and three second biometric data are used. Referring to FIG. 13, in order to obtain the third variable sampling rate, the first biometric data, which is the glucose data, the second biometric data, which is the body temperature data, the third biometric data, which is the heart rate data, and the fourth biometric data, which is the exercise amount data, may be used.

The process of obtaining the first variable sampling rate and the second variable sampling rate may be obtained using various embodiments described above, and a detailed description thereof will be omitted (S1030 and S 1060).

The first variable sampling rate related to the glucose data has a value that increases the first sampling rate by the obtained increment. The second variable sampling rate related to the body temperature data has a value that increases the first sampling rate by the obtained increment. The second variable sampling rate related to the heart rate data has a value that increases the first sampling rate by the obtained increment. The second variable sampling rate related to the exercise amount data has a value that decreases the first sampling rate by the obtained decrement.

In this case, the third variable sampling rate may be determined using all of the obtained first variable sampling rate and second variable sampling rate (S1065). For example, weights may be applied to the first variable sampling rate related to the glucose data, the second variable sampling rate related to the body temperature data, the second variable sampling rate related to the heart rate data, and the second variable sampling rate related to the exercise amount data, respectively, and the third variable sampling rate may be determined using the weighted values. As an example, the sampling rate determination unit 150 may acquire the weighted values according to adding up, averaging, or a set algorithm to determine the third variable sampling rate.

When using the apparatus for controlling biometric data and the method of controlling biometric data according to the embodiment of the present invention, the sampling rate varies according to the user's physiological state and activity level, thereby performing the data control suitable for the user's situation. When using the above method, the apparatus for controlling biometric data may reduce power consumption of sensing devices, etc., by decreasing the sampling rate when the user's current state is a low activity state such as sleeping. On the other hand, the apparatus for controlling biometric data may detect rapidly changing biometric information (e.g., glucose value) with high precision by increasing the sampling rate when a user is in an active state, and the user may obtain more accurate and detailed information.

Meanwhile, any of the embodiments described through the drawings above may be used alone, but are not limited thereto. The above embodiments may be combined or applied by a person skilled in the art.

The term '~ unit' used in the present embodiment refers to software or a hardware component such as a field programmable gate array (FPGA) or an application specific integrated circuit (ASIC), and '~ unit' play certain roles. However, '~ unit' is not limited to the software or the hardware. The "-unit" may be configured to be stored in a storage medium that can be addressed or may be configured to regenerate one or more processors. Accordingly, as an example, the "-unit" refers to components such as software components, object-oriented software components, class components, and task components, processes, functions, attributes, procedures, subroutines, segments of program code, drivers, firmware, microcode, circuits, data, databases, data structures, tables, arrays and variables. Components and functions provided within "-unit" may be combined into a smaller number of components and "-unit" or may be further separated into additional components and "-unit." Furthermore, components and '~ units' may be implemented to reproduce one or more central processing units (CPUs) in a device or a security multimedia card.

Although the above description has been made based on the embodiments, this is only an example and does not limit the present invention. Those skilled in the art to which the present invention pertains may understand that several modifications and applications that are not described in the present specification may be made without departing from the spirit of the present invention. For example, each component described in detail in the embodiment may be modified and implemented. In addition, differences associated with these modifications and applications are to be interpreted as falling within the scope of the present invention as defined by the following claims.

### [Detailed Description of Main Elements]

10: Sensor transmitter
20: Receiver
100: Apparatus for controlling biometric data
110: Obtaining unit
120: Indicator acquisition unit
130: Comparison unit
140: Result obtaining unit
150: Sampling rate determination unit
160: Control unit

## Claims

1. A method of controlling biometric data, comprising:
obtaining a first biometric data collected according to a first sampling rate;
acquiring at least one first indicator value related to a first time interval of the first biometric data;
acquiring at least one second indicator value related to a second time interval of the first biometric data;
obtaining a first comparison result based on ( i ) a difference value between the at least one first indicator value and the at least one second indicator value and (ii) threshold values set related to the first indicator value and the second indicator value;
obtaining a first adjustment result for the first sampling rate based on the first comparison result; and
determining a first variable sampling rate for the first biometric data based on the first adjustment result.

2. The method of claim 1, wherein the first time interval is arranged with priority over the second time interval.

3. The method of claim 1, wherein the obtaining of the first comparison result based on the set threshold value includes:
determining whether a size of the difference value is greater than the threshold value; and
determining a sign of the difference value based on that the size of the difference value is greater than the threshold value.

4. The method of claim 3, wherein the obtaining of the first adjustment result for the first sampling rate includes obtaining the first adjustment result as a adjustment result which maintains the first sampling rate based on that the size of the different value is less than or equal to the threshold value.

5. The method of claim 4, wherein the obtaining of the first adjustment result for the first sampling rate includes obtaining the first adjustment result as a adjustment result which increases the first sampling rate based on that the difference value is greater than the threshold value and the sign of the difference value is a first sign,.

6. The method of claim 5, wherein the obtaining of the first adjustment result for the first sampling rate includes obtaining the first adjustment result as a adjustment result which decreases the first sampling rate based on that the difference value is greater than the threshold value and the sign of the difference value is a second sign.

7. The method of claim 6, wherein, in the obtaining the first adjustment result for the first sampling rate, at least one of an increment and a decrement of the first sampling rate is determined based on the size of the difference value.

8. The method of claim 1, wherein the obtaining of the at least one second biometric data includes:
acquiring at least one third indicator value related to the first time interval of each of the at least one second biometric data;
acquiring at least one fourth indicator value related to the second time interval of each of the at least one second biometric data;
obtaining a second comparison result based on ( i ) a difference value between the at least one third indicator value and the at least one fourth indicator value and (ii) threshold values set related to the third indicator value and the fourth indicator value;
obtaining a second adjustment result for the first sampling rate based on the second comparison result; and
determining a second variable sampling rate for the first biometric data based on the second adjustment result.

9. The method of claim 8, comprising:
determining a third variable sampling rate for the first biometric data based on ( i ) the first variable sampling rate determined based on the first biometric data and (ii) at least one second variable sampling rate obtained based on the at least one second biometric data.

10. The method of claim 9, wherein the determining of the third variable sampling rate includes determining the third variable sampling rate applied a weight related to each of the first variable sampling rate determined based on the first biometric data and the at least one second variable sampling rate determined based on the at least one second biometric data.

11. The method of claim 9, wherein, in the determining of the third variable sampling rate,
a set number of variable sampling rates among the first variable sampling rate determined based on the first biometric data and the at least one second variable sampling rate determined based on the at least one second biometric data are selected according to a preset condition, and
the third variable sampling rate is determined based on the selected variable sampling rate.

12. The method of claim 9, comprising:
controlling to collect the first biometric data according to the first variable sampling rate or the third variable sampling rate.

13. An apparatus for controlling biometric data, comprising:
an obtaining unit that obtains first biometric data collected according to a first sampling rate;
an indicator acquisition unit that acquires at least one first indicator value related to a first time interval of the first biometric data and acquires at least one second indicator value related to a second time interval of the first biometric data;
a comparison unit that obtains a first comparison result based on ( i ) a difference value between the at least one first indicator value and the at least one second indicator value and (ii) threshold values set related to the first indicator value and the second indicator value;
a result obtaining unit that obtains a first adjustment result for the first sampling rate based on the first comparison result; and
a sampling rate determination unit that determines a first variable sampling rate for the first biometric data based on the first adjustment result.

14. The apparatus of claim 13, wherein the obtaining unit obtains at least one second biometric data,
the indicator acquisition unit acquires at least one third indicator value related to the first time interval of each of the at least one second biometric data and acquires at least one fourth indicator value related to the second time interval of each of the at least one second biometric data,
the comparison unit obtains a second comparison result based on ( i ) a difference value between the at least one third indicator value and the at least one fourth indicator value and (ii) threshold values set related to the third indicator value and the fourth indicator value,
the result obtaining unit obtains a second adjustment result for the first sampling rate based on the second comparison result, and
the sampling rate determination unit determines a second variable sampling rate for the first biometric data based on the second adjustment result.

15. The apparatus of claim 14, comprising:
a control unit that controls to collect the first biometric data according to the first variable sampling rate or the third variable sampling rate,
wherein the sampling rate determination unit determines a third variable sampling rate for the first biometric data based on ( i ) the first variable sampling rate determined based on the first biometric data and (ii) at least one second variable sampling rate determined based on the at least one second biometric data.
